(19) Europäisches Patentamt
European Patent Office
Office européen des brevets

(11) Publication number: 0 042 279
B1

# (12) EUROPEAN PATENT SPECIFICATION

(45) Date of publication of patent specification: 13.11.85

(21) Application number: 81302660.6

(22) Date of filing: 15.06.81

(51) Int. Cl.[4]: C 07 D 239/16, C 07 D 243/04, A 61 K 31/505, A 61 K 31/55

(54) **N-aryl-N'((1,4,5,6-tetrahydropyrimidin-2-yl) or (4,5,6,7-tetrahydro-1-H-1, 3-diazepin-2-yl)ureas for intestinal disorders and as antihypertensives.**

(30) Priority: 16.06.80 US 159988
16.06.80 US 159986
16.06.80 US 159987
16.06.80 US 159985

(43) Date of publication of application:
23.12.81 Bulletin 81/51

(45) Publication of the grant of the patent:
13.11.85 Bulletin 85/46

(84) Designated Contracting States:
AT BE CH DE FR GB IT LI NL SE

(56) References cited:
US-A-3 168 520
US-A-4 204 000

Arzneimittel-Forschung, Vol. 28 (II), supplement 8a,1978 Aulendorff/Württ. G.H.Douglas et al. "Antimotility and Antisecretory Activity of some Aryl Substituted Amidinoureas" pages 1435-1441.

(73) Proprietor: McNeilab, Inc.
Camp Hill Road
Fort Washington Pennsylvania 19034 (US)

(72) Inventor: Rasmussen, Chris Royce
1540 Butler Pike
Ambler Pennsylvania 19002 (US)

(74) Representative: Jones, Alan John et al
CARPMAELS & RANSFORD 43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 042 279 B1

**Description**

*Field of Invention*

This invention relates to compounds, for controlling hypertension, relieving the symptoms associated with irritable bowel syndrome and controlling diarrhea, which are certain *N*-aryl-*N'*-[(1,4,5,6-tetrahydropyrimidin-2-yl) or (4,5,6,7-tetrahydro-1-H-1,3-diazepin-2-yl)]ureas, to processes for producing said urea compounds and to pharmaceutical compositions containing said urea compounds.

*Background of the Invention*

Irritable bowel syndrome is a functional disorder characterized primarily by abdominal pain, but also by diarrhea and constipation and like symptoms. It is believed that the pain associated with this syndrome is due to excessive sensitivity to distention of the bowel caused either by intestinal gas and/or fecal material. Treatments which would provide relief from the discomforts associated with such symptoms and/or the disorder which produce such symptoms are highly desirable.

*Brief Summary of the Invention*

The present invention provides compounds for reducing arterial pressure in hypertensive animals, for relieving the symptoms associated with irritable bowel syndrome and for controlling diarrhea in animals having said syndrome or in animals having diarrhea, respectively. The compounds are used by administering to said animal in a pharmaceutically-acceptable carrier, a therapeutically-effective amount of the compound, or a pharmaceutically-acceptable salt thereof.

The compounds of the present invention have the formula:

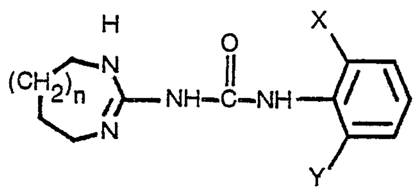

(I)

wherein X is Br or Cl, and Y is Br, Cl or $CH_3$; and n is 0 or 1.

Preferred compounds are those wherein X and Y are each Cl, which is the most active compound for the above methods involving intestinal disorders, and its pharmaceutically-acceptable salts, and wherein X is Cl, and Y is $CH_3$, and its salts.

This invention also embraces compositions, suitable for use in treating hypertension, wherein the foregoing urea compound is in admixture with a pharmaceutically acceptable carrier. The most preferred composition contains the compound wherein X and Y are each Cl, which is the most active antihypertensive compound, or its pharmaceutically-acceptable salts.

The activities of the above compounds reside in the urea base so that useful acid addition salts may be from various acids provided only that the acids be pharmaceutically-acceptable. Representative acid salts include hydrochloride, hydrobromide, phosphate, sulfate, *p*-toluenesulfonate, benzenesulfonate, malonate, succinate, methosulfate, methanesulfonate and 2-napsylate.

*Prior Art*

Certain N-(aryl)-tetrahydropyrimidin-2-yl ureas are generically disclosed in U.S. Patent No. 3,168,520, but the only utility taught is in connection with dyeing, and no pharmaceutical utility is taught, nor are any of the compounds used in the present invention specifically disclosed.

An article by G. H. Douglas, et al., *Arzneimittel Forschung.,* Vol. 28 (II) Supplement 8a, 1435—1441 (1978) discloses as Compound 109 at p. 1438 (in its tautomeric form) the compound of the formula:

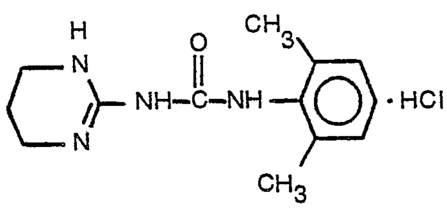

which was disclosed to be active as a gastric acid antisecretory agent. The article does not teach the antihypertensive activity, the activity as an antidiarrheal agent or in relieving the symptoms associated with irritable bowel syndrome for this 2,6-dimethylphenyl compound, which applicant found to exist.

*Prior Art*

The above article by G. H. Douglas, et al., *Arzneimittel Forschung.,* Vol. 28 (II) Supplement 8a,

1435—1441 (1978) erroneously discloses as Compound 110 at p. 1438 (in its tautomeric form) the compound of the formula:

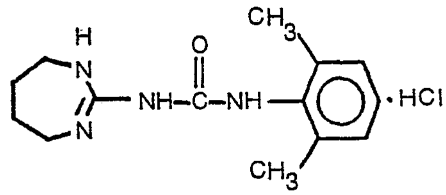

which was disclosed to be active as a gastric acid antisecretory agent. The article does not teach the antihypertensive activity, the activity as an anti-diarrheal agent or in relieving the symptoms associated with irritable bowel syndrome for this 2,6-dimethylphenyl compound, which applicant found to exist.

The columns headed by $R_2$ and $R_3$ in Table 3 on p. 1438 of said article were transposed erroneously leading to the above structure, which was then disclosed in *Chem. Abstracts, 90,* 48236s (1979), where it was named in the *Chem. Abstracts,* Vol. *90,* 1979—Formula Index and given a Chem. Abstracts Registry Number.

*Method of Preparation*

The pharmacologically useful *N*-aryl-*N'*-(1,4,5,6-tetrahydropyrimidine-2-yl)urea compounds are prepared from 2-amino-1,4,5,6-tetrahydropyrimidine·HCl, which has the structure:

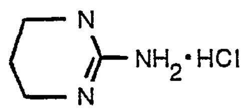 (II)

and which is a known compound (R. F. Evans and D. J. Brown, *J. Chem. Soc.,* 4039 (1962) prepared according to the following reaction scheme:

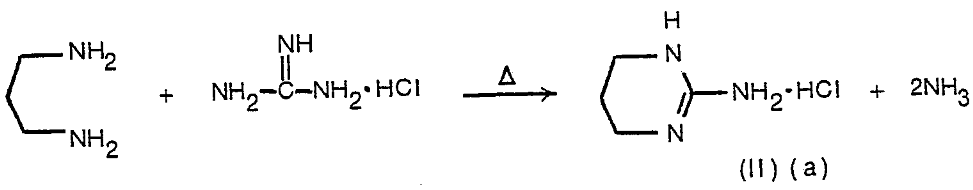


(II) (a)

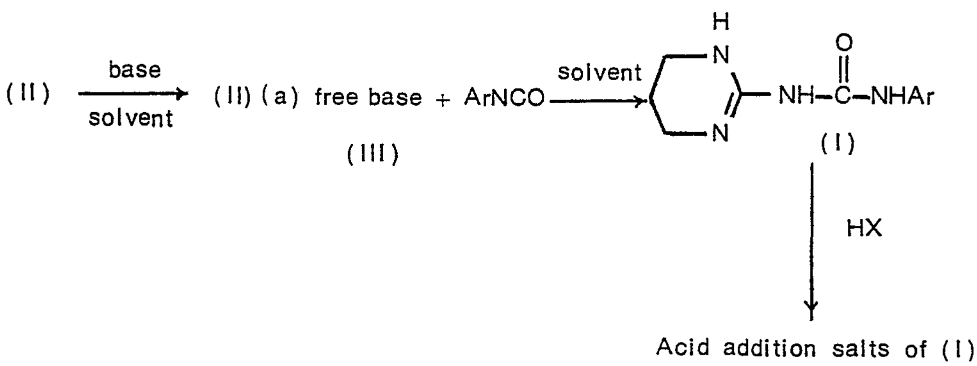


wherein Ar is 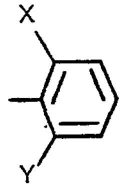, and X and Y are as defined above.

The pharmacologically useful *N*-aryl-*N'*-(4,5,6,7-tetrahydro-1*H*-1,3-diazepin-2-yl)urea compounds are prepared from 2-amino-4,5,6,7-tetrahydro-1*H*-1,3-diazepine II, which has the structure:

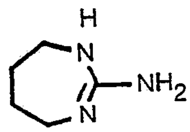

(II) (b)

and which is a known compound.

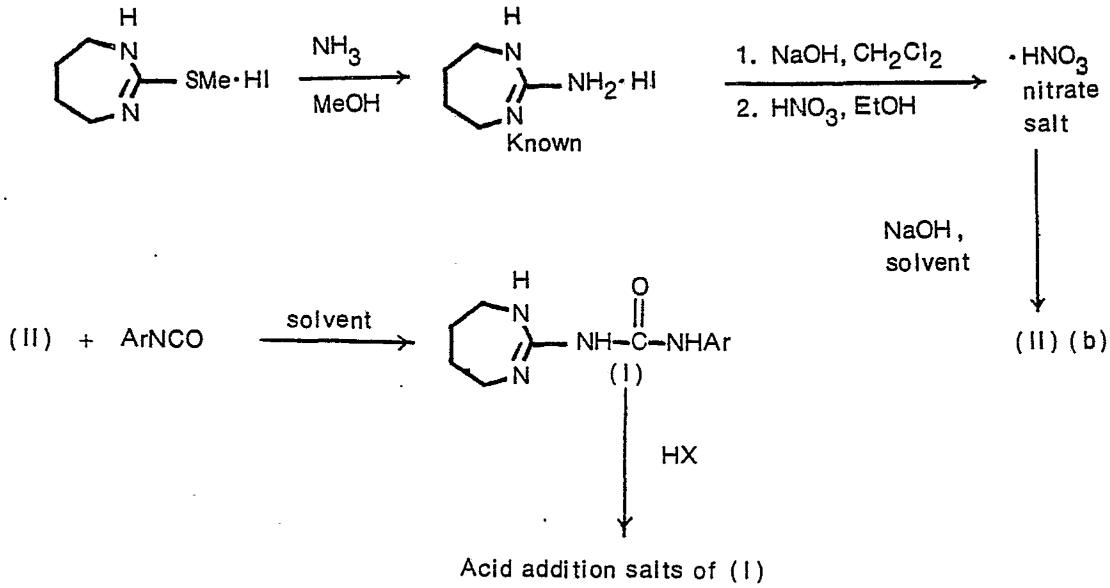


wherein Ar is as defined above.

The free base form of II can be generated by treating a stirred suspension of II HCl in solvents such as $CH_2Cl_2$-(methylene chloride), tetrahydrofuran and dioxane with strong bases such as aqueous NaOH (50%) and concentrated KOH, NaOH (50%) being preferred. The resulting solution of free base II is dried over a suitable drying agent such as $Na_2SO_4$ or $K_2CO_3$. The dried solutions may either be used as such for reactions with aryl isocyanates to obtain products I or the extraction solvent may be evaporated *in vacuo* and the residual free base II taken up in a different organic solvent, e.g. DMF or DMSO, said solutions of II then being treated with an appropriate isocyanate III to afford the free base products I. Although the reactions of II with III may be carried out with equimolar amounts of reactants, usually a stoichiometric excess, generally of from 0.25—1.0 mole of free base II to that of aryl isocyanate III is employed in order to minimize undesired side reactions, such as formation of bis-aryl isocyanate adducts with II. Temperature for those reactions may conveniently range from −20° to 70°C. The products I, obtained in free base form, may conveniently be purified by dissolving in an organic solvent, immiscible with $H_2O$, such as $CH_2Cl_2$, washing with $H_2O$ to remove excess II, if any, followed by isolation of I from the solvent by drying, filtration from drying agent, and solvent removal.

Alternatively, the reactions of II free base with III may be carried out by adding a solution of II HCl in DMF or DMSO to a stirred suspension of a stoichiometric amount of an alkali metal hydride such as LiH or NaH, LiH being preferred, which forms II free base, the corresponding alkali metal chloride, and $H_2$ gas.

The thus obtained solution of free base II (the presence of the metal chloride does not interfere with the subsequent reaction) is treated with an appropriate amount of aryl isocyanate III. When the reaction is complete, dilution with $H_2O$ or ice-$H_2O$, in excess amounts, causes precipitation of crude product I and leaves any unreacted II in solution. Filtration then allows isolation of crude I.

Said I, in free base form, may be further purified, if necessary, for instance by recrystallization or chromatographic techniques, according to standard techniques known in the art. A further purification method may be used such as dissolution in dilute aqueous acid, such as HCl, most preferred, $H_2SO_4$, HBr or $HNO_3$, filtration from any undissolved impurities, followed by neutralization with suitable inorganic bases, such as sodium and potassium bicarbonates and carbonates, dilute alkali metal hydroxides such as NaOH or KOH, and organic bases such as triethylamine or diisopropylethylamine, which causes precipitation of I free base. The thus-obtained free base I may then be purified, by for instance recrystallization, as described above, or may be converted to a suitable pharmaceutically-acceptable salt form of I which also may be purified by recrystallization or precipitation techniques well known in the art.

Said pharmaceutically-acceptable salt forms of I are generally comprised of I in combination with suitable mineral acids such as HCl (most preferred), HBr, $H_2SO_4$, $H_3PO_4$, and strong organic acids such as benzenesulfonic, *p*-toluenesulfonic, 1- and 2-naphthalenesulfonic, ethanedisulfonic or methane- and ethanesulfonic; methylsulfonic being the most preferred. Although salts of I with weaker acids, such as

benzoic, fumaric, maleic or citric do form, they are relatively easily dissociated because of the relatively weak base strength of I. This dissociation may be caused by attempted drying *in vacuo* or dissolution in $H_2O$. The ease of dissociation, however, may not necessarily preclude use of salts of this type in pharmaceutical formulations insofar as they remain stable enough to be purified by for instance recrystallization and capable of being formulated into pharmaceutical preparations such as tablets or capsules.

The preferred salt forms of I are additionally capable of forming hydrates and solvates with $H_2O$ and certain organic solvents, respectively. Also, I and its salt forms may exist in several tautomeric forms. It is naturally intended that the various hydrates, solvates, and tautomeric forms of I be included within the scope of this invention.

The *N*-aryl-*N'*-[(1,4,5,6-tetrahydropyrimidin-2-yl) or (4,5,6,7-tetrahydro-1-H-1,3-diazepin-2-yl)]urea compounds of the present invention have been found to be useful as anti-diarrheal agents and to provide relief from the discomfort associated with functional intestinal disorders known as irritable bowel syndrome. The disorder is characterized by abdominal pain, diarrhea, constipation and like symptoms caused by excessive sensitivity to distension of the bowel by the presence of intestinal gas and/or fecal material.

The *N*-(substituted phenyl)-*N'*-[(1,4,5,6-tetrahydropyrimidin-2-yl) or (4,5,6,7-tetrahydro-1-H-1,3-diazepin-2-yl)] urea compounds of the present invention have been found to alleviate hypertension and further, to generally accomplish this without an accompanying increase in heart rate. The most useful compounds of the present invention do not show an increase in heart rate, but a lowering of heart rate and, generally, long duration. An agent which has an antihypertensive effect without increasing but rather maintaining or decreasing heart rate, is the one considered most useful for beneficially treating a hypertensive subject. The extent to which a compound possesses these properties may be primarily determined in the antihypertensive test hereinafter described.

*Rodent Antihypertensive Screen* — This test evaluates compounds for effects on arterial pressure and heart rate. In this test, the arterial pressure of adult spontaneously hypertensive rats [SHR] (Charles River) is monitored directly via an aortic cannula. Rats are anesthetized with an inhalation anesthetic (methoxyflurane). The left carotid artery is isolated and cannulated. The tip of the cannula is advanced to the aorta and the cannula is exteriorized behind the neck at the level of the scapula. Animals are placed in individual cases and allowed to recover from the anesthetic and are kept unrestrained. The arterial cannula is connected to a pressure transducer which is attached to a recorder. Heart rate is determined from the arterial pressure recording. The test compounds are administered either orally (p.o.) by gavage or by intraperitoneal (i.p.) injection. The arterial pressure and heart rate are monitored for a minimum of 24 hours. A test compound is considered to be active as an antihypertensive agent if the mean arterial pressure (MAP) indicates a fall of >20 mbar (>15 mm of Hg.). Each animal serves as his own control.

The results of this test employing at least 3 rats per dose level for each compound and performed with *N*-aryl-*N'*-(1,4,5,6-tetrahydropyrimidin-2-yl))urea compounds are shown in Table I.

The results seen in Tables 1A and 1B show that *N*-(substituted phenyl)-*N'*-[(1,4,5,6-tetrahydropyrimidin-2-yl) or (4,5,6,7-tetrahydro-1-H-1,3-diazepin-2-yl)]urea compounds and their salts possess not only the beneficial antihypertensive property but also the desirable property of maintaining or lowering heart rate.

The compounds of the present invention are useful for treating hypertension (high blood pressure) by administering to subjects in need of treatment, a therapeutically-effective hypertension reducing amount of a *N*-(substituted phenyl)-*N'*-[(1,4,5,6-tetrahydropyrimidin-2-yl) or (4,5,6,7-tetrahydro-1-H-1,3-diazepin-2-yl)]urea compound of Formula I or its pharmaceutically-acceptable salt as active agent. The active agents may be administered with or without carrier in the amounts hereinafter set forth. A preferred method of administration is by the use of pharmaceutical compositions in unit dosage form as described below.

The operable ranges for carrying out the treatment is, for administration, orally or parenterally, from 1 milligram to 500 milligrams of said *N*-(substituted phenyl)-*N'*-[(1,4,5,6-tetrahydropyrimidin-2-yl) or (4,5,6,7-tetrahydro-1-H-1,3-diazepin-2-yl)]urea compound in dosage unit form. While the therapeutic compounds are most useful for human subjects, they may also be employed for other mammals. Operable amounts are generally within the range of from 0.5 to 100 mg/kg of body weight.

TABLE 1–A

ANTIHYPERTENSIVE AND CARDIAC RATE DETERMINATIONS

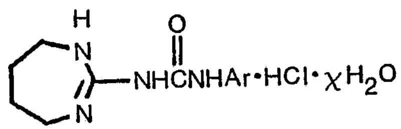

| | | | SHR Rat | | |
|---|---|---|---|---|---|
| Ar | McN– | X | MAP in Δmm Hg (mg/kg, route) | ΔHR in beats/min. | Duration in hours |
| 2,6–$Cl_2$Ph | 4951(base) | – | –54 (35 p.o.) | –86 | 21 |
| 2,6–$Cl_2$Ph | 4951(base) | – | –79 (100 p.o.) | –114 | >24 |
| 2,6–$Cl_2$Ph | 4951(base) | – | –58 (30 p.o.) | –140 | >24 |
| 2,6–$Cl_2$Ph | 4951(base) | – | –78 (30 i.p.) | –100 | >24 |
| 2,6–$Cl_2$Ph | 4951(base) | – | –41 (10 p.o.) | –76 | >24 |
| 2,6–$Cl_2$Ph | 4951(base) | – | –42 (3 p.o.) | –36 | 17 |
| 2,6–$Cl_2$Ph | 4951(base) | – | –24 (1 p.o.) | –44 | 1 |
| *2–$CF_3$Ph | 5027–11–98 | 1 | –49 (30 i.p.) | –110 | 11 |
| | | | –65 (100 p.o.) | –74 | 11 |
| *2,6–$Me_2$Ph | 5028–11–98 | 1.1 | –70 (30 i.p.) | –126 | 23 |
| | | | –82 (100 p.o.) | –168 | >24 |
| 2–Cl–6–MePh | 5058–11–98 | 0.5 | –73 (30 i.p.) | –103 | >24 |
| | | | –66 (100 p.o.) | –118 | >24 |
| *2,6–$Br_2$–4–FPh | 5042–11 | 0 | –90 (30 i.p.) | –60 | 17 |
| | | | –37 (100 p.o.) | –71 | 11 |

MAP = Mean Arterial Pressure

Δ = Change

HR = Heart Rate

* = Compounds not according to the invention

TABLE 1–B

ANTIHYPERTENSIVE AND CARDIAC RATE DETERMINATIONS

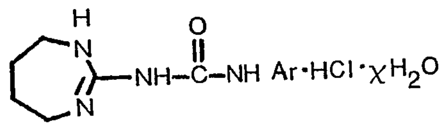

| | | | SHR Rat | | |
|---|---|---|---|---|---|
| Ar | χ | McN– | ΔMAP in mm Hg (mg/kg, route) | ΔHR in beats/min. | Duration in Hours |
| 2,6–$Cl_2$Ph | 0.5 | 4967–11–98 | –49 (35 p.o.)<br>–50 (30 p.o.)<br>–48 (10 p.o.)<br>–31 (1 p.o.) | –78<br>–124<br>–67<br>–64 | 21<br>>24<br>23<br>17 |
| *2–$CF_3$Ph | 0 | 5036–11 | –43 (30 i.p.)<br>–39 (100 p.o.) | +39<br>–98 | <1<br>11 |
| 2,6–$Me_2$Ph | 1/3 | 5076–11–98 | –94 (30 i.p.)<br>–64 (100 p.o.) | –127<br>–112 | >24<br>>24 |
| 2–Cl–6–MePh | 1/3 | 5075–11–98 | –81 (30 i.p.)<br>–58 (100 p.o.) | –108<br>–104 | 17<br>23 |
| *2,6–$Br_2$–4–F Ph | 0 | 5037–11 | –30 (30 i.p.)<br>–43 (100 p.o.) | +58<br>+41 | 1<br>23 |
| *2,6–$(OMe)_2$Ph | 0 | 5040–11 | –87 (30 i.p.)<br>–79 (100 p.o.) | +62<br>–50 | 1<br>11 |

MAP = Mean arterial pressure

Δ = Change

HR = Heart Rate

* Compounds not according to the invention

*Definition of "irritable bowel syndrome"* — this condition, which is also known as irritable colon, spastic colon or mucous colitis, is a clinical disorder characterized by either diarrhea or constipation, in association with abdominal discomfort, but without demonstrable structural bowel disease. It is classified as a functional bowel disease. In practice, it is one of the major digestive disorders and is a leading cause of absenteeism from work and a reason for physician office visits. In modern usage, "irritable bowel syndrome" is the preferred term for this condition.

*Pathophysiology* — Although the etiology is unknown, recent studies have elucidated characteristics of the pathophysiology of this condition. Alteration of colonic motility has been suggested as a mechanism of this syndrome. This is characterized by an increased basal activity, as well as a sensitivity to exogenous or endogenous stimuli. Since the symptoms many times are brought on by eating, it has been postulated that cholecystokinin, which is released from the small intestine after eating is partially responsible.

Electrical recordings from the human colon have also shown that there is a difference in the characteristic slow wave frequency in patients with irritable bowel syndrome.

Patients with irritable bowel syndrome have a lower pain threshold to distention than normal. Thus, they are much more sensitive to the distention caused by normal amounts of gas or fecal contents present in the colon. If the sensitivity to distention can be reduced, the pain associated with irritable bowel syndrome necessarily will be reduced.

Irritable bowel syndrome appears to be unique to man and no naturally-occurring disease similar to irritable bowel syndrome has been discovered in animals. In order to test compounds for possible activity in this state, the pathophysiologic characteristics of the disease must be utilized in designing animal experiments to test such compounds. The "glass bead test" described below has been developed to test for the effectiveness of compounds against one component of this disease state, that of response of the colon to distention, which could produce abdominal pain. Although no way of producing an increased sensitivity to distention of the bowel has been found in the mouse, stimulation by a greater than normal distending force is utilized. A drug interfering with this response to distention may be of use in the treatment of irritable bowel syndrome if it has other characteristics which could provide efficacy.

The "glass bead test" can also be used to evaluate antidiarrheal activity. Although most drugs producing constipation in man are effective in the glass bead test, many work by mechanisms which do not fulfill the characteristics of an agent which both decreases the response to distention and effects abnormal motor activity. One example is that of narcotic analgesics which are effective in the glass bead test, but increase the phasic motor activity of the intestine and are not useful in irritable bowel syndrome. Other test models should, therefore, be used to confirm or further define the utility of any compound as an anti-irritable bowel agent.

One test to confirm the utility of compounds effective in the glass bead test, which may be used as an indicator of effectiveness for the diarrhea state produced by irritable bowel syndrome is the "castor oil diarrhea test" in mice or rats. (A description of this test is given *infra*.) The series of compounds for which there is claimed anti-irritable bowel activity in fact are active against castor oil diarrhea, the effective doses correlating well with those which are effective in the glass bead test.

Since there is no model for the constipation phase of this condition, the regularizing action of a compound cannot be examined. However, since constipation in irritable bowel syndrome is often secondary to diarrhea, one would expect that the prevention of diarrhea should alleviate the constipation phase.

*The Glass Bead Test:* The extent to which a compound is effective in providing relief from the symptoms associated with irritable bowel syndrome may be determined by a test in which a glass bead is inserted into the rectum and the lapse time between insertion and expulsion of the bead determined. Compounds which are effective in decreasing the sensitivity to distention of the colonic wall delays the expulsion of the bead.

The test is carried out with male albino mice of 18—25 grams body weight using groups of five mice for each compound dose tested. The initial screen dose selected for all compounds is 50 milligrams per kilogram of body weight (mg/kg) administered orally in a volume of 0.1 milliliter per 10 grams of body weight. The control groups receive the vehicle, 0.5 per cent methocel, used for both oral and intraperitoneal administration. The mice are fasted one hour before testing and the test drugs are given one hour prior to glass bead insertion.

At the end of the pretreatment time, the mouse is picked up and held firmly in one hand with his abdomen facing the technician. The glass bead of 3 millimeters in diameter is positioned at the rectum and using a pinching action with a thumb and forefinger, the bead is pushed into the rectum. Then using a glass rod of 3 millimeters in diameter which has been lubricated with 0.5 per cent methocel to facilitate insertion, the glass bead is pushed up into the rectum a distance of 2 centimeters using a slow gentle turning motion. The mice are timed as a group using the last mouse inserted as zero time and the number of beads expelled in a group at different timed intervals are recorded. The groups are based on timed intervals of 0 to 5 minutes, 5 to 10 minutes, 10 to 20 minutes, 20 to 40 minutes, and greater than 40 minutes. They are given the activity index values of 0, 1, 2, 3 and 4, respectively. Mice who have not expelled their beads by the 40-minute cutoff time are examined for perforations. Those mice whose colons are perforated are eliminated from the group. The sum of the values divided by the number of mice or heads is termed the activity index for the drug tested. The $ED_{50}$ is determined by regression lines using the method of least squares. The $ED_{50}$

is arbitrarily assigned as that dose causing an activity index of 2. The $ED_2$ is essentially equivalent to the $ED_{50}$, with the activity being expressed on a scale of 0 to 4 in the Glass Bead Test.

The results of this test employing intraperitoneal and oral administration of *N*-aryl-*N'*-[(1,4,5,6-tetrahydropyrimidin-2-yl) or (4,5,6,7-tetrahydro-1-H-1,3-diazepin-2-yl)]urea compounds are shown in Tables 2A and 2B. Tables 2A and 2B also contain results of the Mouse Castor Oil Method Test described below.

TABLE 2–A

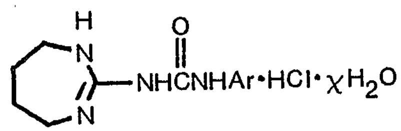

| | | | MOUSE | | |
|---|---|---|---|---|---|
| | | | M = 5–10 Glass Bead Test $ED_2$P.O. mg/kg | M = 5 Castor Oil Diarrhea Test mg/kg p.o. | |
| Ar | χ | McN– | | 1 hour | 5 hours |
| 2,6–$Cl_2$Ph | – | 4951(base) | 9.4 | 1.6 | 3.9($ED_{50}$) |
| 2,6–$Cl_2$Ph | 0 | 4951–11 | 7 mg/kg p.o.<br>4.3 mg/kg i.p. | | 6.5($ED_{50}$) |
| *2–$CF_3$Ph | 1 | 5027–11–98 | >200 | 100 | >100<br>200 lethal (4/5) |
| *2,6–$Me_2$Ph | 1.1 | 5028–11–98 | 28.6 | <50 | 21.5($ED_{50}$) |
| 2–Cl–6–Me Ph | 0.5 | 5058–11–98 | <6.25 | – | 18 |
| *2,6–$Br_2$–4–F Ph | 0 | 5042–11 | >200 | 50 | 145 |

*Compounds not according to the invention.

TABLE 2–B

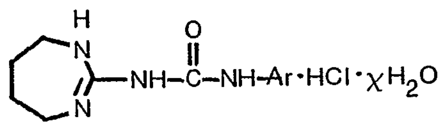

| Ar | χ | McN– | MOUSE: n = 5–10 Glass Bead Test $ED_2$ P.O. mg/kg | MOUSE: 3n=5 Castor Oil Diarrhea mg/kg P.O. 1 hour | MOUSE: 3n=5 Castor Oil Diarrhea mg/kg P.O. 5 hours |
|---|---|---|---|---|---|
| 2,6–$Cl_2$Ph | 0.2 | 4967–11–98 | 9.7 | 6.25 | 10.4 ($ED_{50}$) |
| *2–$CF_3$ Ph | 0 | 5036–11 | 146 | 50 | >100<br>200 lethal (4/5) |
| *2,6–$Me_2$Ph | 1/3 | 5076–11–98 | 22.3 | – | 41 ($ED_{50}$) |
| 2–Cl–6–MePh | 1/3 | 5075–11–98 | 16.2 | – | 26 ($ED_{50}$) |
| *2,6–$Br_2$–4–FPh | 0 | 5037–11 | 91.4 | 50 | 108 ($ED_{50}$) |
| *2,6–$(OMe)_2$Ph | 0 | 5040–11 | 200 | 50 | >100<br>200 lethal (4/5) |

*Compounds not according to the invention.

*Mouse Castor Oil Method Test.* — Male albino mice weighing 18—30 gms are employed for this study. The mice are fasted overnight before testing; however, water is given *ad lib.* during fast. On the day of testing, the mice are weighed beforehand to obtain an average weight using an individual weight range of ±5 gms.

Drugs are administered by the desired route (p.o., s.c., or i.p.) suspended or dissolved in 0.5 per cent methocel using a volume of 1 ml/100 gm of average body weight (0.1 ml/10 gm) to groups of five mice for each compound dose tested. Control mice receive the test vehicle. One hour after, a single dose of castor oil (0.3 ml per mouse) is given orally to all groups. The mice are returned to their cages with access to food and water for the remainder of the experiment.

Mice fasted overnight will have diarrhea usually within one hour after administration of castor oil. Observations for the presence of diarrhea are taken every hour for five hours. A positive or negative response is used. The absence of diarrhea is expressed in percent inhibition as compared to the incidence of diarrhea at that time period in the control group. $ED_{50}$'s are determined by calculation regression analysis using a least square method. The effect over the total duration of the experiment is used for this calculation.

The foregoing results in Tables 2-A and 2-B illustrate the beneficial effect of *N*-aryl-*N'*-[(1,4,5,6-tetrahydropyrimidin-2-yl) or (4,5,6,7-tetrahydro-1-H-1,3-diazepin-2-yl)]urea compounds in decreasing abnormal sensitivity to distention of bowel. These properties demonstrate the utility of the compounds and compositions of the present invention.

The compounds of the present invention are used in a method for alleviating functional intestinal disorders and for treating the discomforts associated therewith by administering to subjects in need of treatment, a therapeutically-effective amount of a compound of Formula I or its pharmaceutically-acceptable salt as active agent. The active agents may be administered with or without carrier in the amounts hereinafter set forth. A preferred method of administration is by use of pharmaceutical compositions in unit dosage form as described below.

The operable ranges for carrying out the treatment is, for administration, orally or parenterally, from 1 milligram to 500 milligrams of an *N*-aryl-*N'*-[(1,4,5,6-tetrahydropyrimidin-2-yl) or (4,5,6,7-tetrahydro-1-H-1,3-diazepin-2-yl)]urea compound in dosage unit form. While the therapeutic compounds are most useful for human subjects, they may also be employed for other mammals. Operable amounts are generally within the range of from 0.50 to 100 mg/kg.

The outstanding properties are most effectively utilized by use of the pharmaceutical compositions of the present invention. The pharmaceutical compositions comprising an *N*-aryl-*N'*-(1,4,5,6-tetrahydropyrimidin-2-yl)urea compound or acid addition salt thereof, as the active ingredient, may be prepared by intimately mixing the urea compound with a pharmaceutical carrier according to conventional pharmaceutical compounding techniques, which carrier may take a wide variety of forms depending on the form of preparation desired for administration, e.g. oral or parenteral. In preparing the compositions in oral dosage form, any of the usual pharmaceutical media may be employed. Thus, for liquid oral preparations, such as suspensions, elixirs and solutions, suitable carriers and additives include water, glycols, oils, alcohols, flavoring agents, preservatives and coloring agents; for solid oral preparations such as powders, capsules and tablets, suitable carriers and additives include starches, sugars, diluents, granulating agents, lubricants, binders and disintegrating agents. Because of their ease in administration, tablets and capsules represent the most advantageous oral dosage unit form, in which case solid pharmaceutical carriers are employed. If desired, tablets may be sugar-coated or enteric-coated by standard techniques. For parenterals, the carrier will usually comprise sterile water, though other ingredients, for example for aiding solubility or for preservation, may be included. Injectable suspensions may also be prepared, in which case appropriate liquid carriers or suspending agents may be employed. The term "dosage unit form" as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets, capsules, pills, powder packets, wafers, teaspoonsful and tablespoonsful and segregated multiples thereof. A dosage unit generally will contain from 10 to 500 mg of the *N*-aryl-*N'*-(1,4,5,6-tetrahydropyrimidin-2-yl)urea compounds, for use in intestinal disorders, and from 1 to 500 mg of said compounds for use an antihypertensives.

The following examples illustrate the preparation of the urea compounds of the invention, but are not to be construed as limiting.

Example 1—A

*N*-(2,6-Dichlorophenyl)-*N'*-(1,4,5,6-tetrahydropyrimidin-2-yl)urea

A solution of 4.20 (0.0424 mol) of 2-aminotetrahydropyrimidine in 30 ml of dry DMF was warmed to 70°C and with stirring there was added 7.16 g (0.0381 mol) of 2,6-dichlorophenyl isocyanate in 25 ml of dry DMF over a period of 0.5 hours. After stirring for 2 hours at 70°C, the reaction mixture was cooled, diluted with $H_2O$ and filtered. The filter cake was washed well with $H_2O$ and air dried. The crude product, 5.1 g (47%), was dissolved in $CH_2Cl_2$, dried over $K_2CO_3$, filtered through a prewashed pad of filter aid and hexane was added to the cloud point. The resulting crystals were filtered, washed with hexane and dried *in vacuo* for 3 hours (50°C, 5 mm Hg) to give pure *N*-(2,6-dichlorophenyl)-*N'*-(1,4,5,6-tetrahydropyrimidin-2-yl)urea,

4.71 g (43%); m.p. 179—180.5°C, TLC: 5×20 cm silica gel; toluene-ether-MeOH (8:4:1) or $CHCl_3$—MeOH—$NH_4OH$ (90:10:1) indicated homogeneity; IR (KBr) 3421, 1628 $cm^{-1}$; UV max. (MeOH) 222 nm (ε=28300) and 239 nm (inflection) (ε =18,000).

ANAL.

| | | |
|---|---|---|
| Calcd. for $C_{11}H_{12}Cl_2N_4O$: | C, 46.01; H, 4.21; N, 19.51. |
| Found: | C, 45.98; H, 4.23; N, 19.48. |

The starting 2-aminotetrahydropyrimidine was prepared according to the procedure of R. F. Evans, D. J. Brown, *J. Chem. Soc.,* 4039, (1962), which was liberated from the hydrochloride with excess 50% NaOH, extracted with $CH_2Cl_2$, dried ($K_2CO_3$) and evaporated *in vacuo* to an oil.

Example 1—B

*N*-(2,6-Dichlorophenyl)*N'*-(1,4,5,6-tetrahydropyrimidin-2-yl)urea Monohydrochloride

A mixture of 8.50 g (0.0625 mol) of 2-amino-1,4,5,6-tetrahydropyrimidine hydrochloride, 10.0 g (0.12 mol) of 50% NaOH and 70 ml of THF was stirred for 0.5 hours at room temperature and then 10.0 g $Na_2SO_4$ was added. After stirring for an additional 0.5 hours, a solution of 9.4 g (0.050 mol) of 2,6-dichlorophenyl isocyanate in 50 ml of THF was added over a period of 1 hour. After stirring for 1 hour, the reaction mixture was filtered, the filtrate evaporated *in vacuo* and the residue dissolved in 25 ml of 10% HCl and 25 ml of $H_2O$ with warming. After filtration through a pad of diatomaceous earth, chilling in ice gave the crystalline hydrochloride which was recrystallized from MeOH-ether affording 12.40 g. This material was ground to a fine powder and dried to constant weight *in vacuo* (60°C, 5 mm Hg) to give 11.97 (74%) of the title compound; m.p. 215—217°C dec., homogeneous by TLC [5×20 cm silica gel GF $CHCl_3$—MeOH—$NH_4OH$ (90:9:1))]. $^1H$—NMR (DMSO-$d_6$) 1.85 [pentet, (poorly resolved) 2H]; 3.35 (t, J=5HZ, 4H); 7.2—7.7 (m, 3H); 9.16 (broad s, 2H) exchangeable; 10.02 (broad s, 1H) exchangeable, 11.19 (brS, 1H) exchangeable. IR(KBr) 3125, 1717, 1678, 1628 $cm^{-1}$; UV max. (MeOH) 241 infl. (ε=11,200) and 220 nm infl. (ε=23,500).

ANAL.

| | |
|---|---|
| Calcd. for $C_{11}H_{12}Cl_2N_4O\cdot HCl$: | C, 40.83; H, 4.05; N, 17.31. |
| Found: | C, 40.78; H, 4.05; N, 17.30. |

Example 2

*N*-(2-Chloro-6-Methylphenyl)-*N'*-(1,4,5,6-tetrahydropyrimidin-2-yl)urea Monohydrochloride Hemihydrate

A mixture of 8.14 g (0.060 mol) of 2-amino-1,4,5,6-tetrahydropyrimidine hydrochloride, 6.0 g (0.075 mol) of 50% NaOH and 75 ml of THF was stirred for 0.5 hours at room temperature and then 15 g $Na_2SO_4$ was added. After stirring for 0.5 hours, a solution of 6.70 g (0.040 mol) of 2-chloro-6-methylphenyl isocyanate in 50 ml of THF was added over a period of 0.5 hours. After stirring for 1 hour, the reaction mixture was filtered, the filtrate evaporated *in vacuo* and the residue dissolved in 25 ml of 10% HCl and 25 ml of $H_2O$ with warming. After filtration through diatomaceous earth, chilling in ice gave the crystalline hydrochloride which was recrystallized from cold $H_2O$ after treatment with charcoal, affording 7.15 g (59%). This material was dried *in vacuo* (20°C, 5 mm Hg) to constant weight to give 7.11 g (59%) of pure *N*-(2-chloro-6-methylphenyl)-*N'*-(1,4,5,6-tetrahydropyrimidin-2-yl)urea monohydrochloride hemihydrate, m.p. (190) 215—220°C melts, then forms a new solid; m.p. 243—245°C; IR(CHCl3) 3224, 1713, 1677, 1641, 1543 $cm^{-1}$; UV max. (MeOH) 236 (16,200) and 217 nm infl (21,500).

ANAL.

| | |
|---|---|
| Calcd. for $C_{12}H_{15}ClN_4O\cdot HCl\cdot 0{\cdot}5H_2O$: | C, 46.17; H, 5.49; N, 17.94; $H_2O$, 2.89. |
| Found: | C, 46.21; H, 5.53; N, 17.75; $H_2O$, 3.14. |

Example 3

According to the teachings of Examples 1—2, the following compounds are prepared:

1. *N*-(2,6-Dibromophenyl)-*N'*-(1,4,5,6-tetrahydropyrimidin-2-yl)urea and acid addition salts.
2. *N*-(2-Bromo-6-chlorophenyl)-*N'*-(1,4,5,6-tetrahydropyrimidin-2-yl)urea and acid addition salts.
3. *N*-(2-Bromo-6-methylphenyl)-*N'*-(1,4,5,6-tetrahydropyrimidin-2-yl)urea and acid addition salts.

Example 4

1,000 hard gelatin capsules, each containing 200 milligrams of *N*-(2,6-dichlorophenyl)-*N'*-(1,4,5,6-tetrahydropyrimidin-2-yl)urea are prepared from the following formulation:

| | Grams |
|---|---|
| *N*-(2,6-Dichlorophenyl)-*N'*-(1,4,5,6-tetrahydropyrimidin-2-yl)urea | 200 |
| Starch | 250 |
| Lactose | 750 |
| Talc | 250 |
| Calcium Stearate | 10 |

A uniform mixture of the ingredients is prepared by blending and employed to fill two-piece hard gelatin capsules. The capsules are suitable to be orally administered to subjects with functional bowel disorders, and to hypertensive subjects to reduce blood pressure.

Example 5

Gelatin capsules are prepared as described in Example 4 except that in the formulation, (for use in intestinal disorders) 400 grams of *N*-(2,6-dibromophenyl)-*N'*-(1,4,5,6-tetrahydropyrimidin-2-yl)urea is employed as active agent providing capsules containing 400 milligrams of *N*-(2,6-dibromophenyl)-*N'*-(1,4,5,6-tetrahydropyrimidin-2-yl)urea.

Example 6

Gelatin capsules are prepared as described in Example 5 except that *N*-(2-chloro-6-methyl)-*N'*-(1,4,5,6-tetrahydropyrimidin-2-yl)urea is employed as the active agent.

Example 7

1,000 compressed tablets, each containing 500 milligrams of *N*-(2-chloro-6-methylphenyl)-*N'*-(1,4,5,6-tetrahydropyrimidin-2-yl)urea are prepared from the following formulation:

| | Grams |
|---|---|
| *N*-(2-Chloro-6-methylphenyl)-*N'*-(1,4,5,6-tetrahydropyrimidin-2-yl)urea | 500 |
| Starch | 750 |
| Dibasic calcium phosphate hydrous | 5,000 |
| Calcium stearate | 2.5 |

The finely powdered ingredients are mixed well and granulated with 10 per cent starch paste. The granulation is dried and compressed into tablets using starch as a disintegrant and calcium stearate as a lubricant.

Example 8

Gelatin capsules are prepared as described in Example 7 except that *N*-(2,6-dibromophenyl)-*N'*-(1,4,5,6-tetrahydropyrimidin-2-yl)urea is employed as the active agent.

Example 9—A

2-Amino-4,5,6,7-Tetrahydro-1*H*-1,3-Diazepine Mononitrate

A suspension of 217.9 g 2-(methylthio)-4,5,6,7-tetrahydro-1*H*-1,3-diazepine hydroiodide in 400 ml of MeOH was treated with anhydrous $NH_3$ (NaOH traps + NaOCl traps) vigorously for 1 hour. All the solid dissolved. The solution was saturated with $NH_3$ and let stand over the weekend at room temperature. TLC showed the reaction to be essentially complete. The MeOH was boiled off, replaced with *t*-BuOH, then cooled and $Et_2O$ added gave the HI salt (hygroscopic). The material was converted to the free base in $CH_2Cl_2$. The organic layer was dried ($K_2CO_3$), filtered and the solvent removed *in vacuo* affording a heavy oil. The oil was taken up in absolute EtOH and carefully neutralized with $HNO_3$ (conc. aqueous). A little solid impurity precipitated, early on, which should have been removed and discarded. Before the acid had all been added, the mixture became too thick to mix thoroughly. A first crop of the salt was collected and

washed with fresh EtOH (95%). The filtrate was then neutralized to pH7 affording a second crop. Recrystallization of these crops was a long and tedious process using MeOH-EtOH (95%) then boiling off the MeOH and filtering hot from less soluble inorganic impurities.

After many recrystallizations, a crop of quite pure product was obtained, 24.7 g (12.8%); m.p. 165—167°C, less pure crop; m.p. 156—161.5°C, 20.9 g (10.8%); IR (KBr) 3251, 3108, 1660, 1598 $cm^{-1}$; NMR. ANAL.

| | |
|---|---|
| Calcd. for $C_5H_{11}N_3 \cdot HNO_3$ (113.16/176.175): | C, 34.09; H, 6.87; N, 31.80. |
| Found: | C, 34.28; H, 6.36; N, 31.59. |

Example 9—B

*N*-(2,6-Dichlorophenyl)-*N'*-(4,5,6,7-tetrahydro-1*H*-1,3-diazepin-2-yl)urea Monohydrochloride Hemihydrate

A 300 ml flask was charged with 12.1 g (0.05 mol) of 2-amino-4,5,6,7-tetrahydro-1*H*-1,3-diazepine hydroiodide 1), 10.0 g of 50% NaOH and 70 ml of THF. After stirring at room temperature for 1 hour, 10.0 g $Na_2SO_4$ was added and the mixture was stirred for an additional 1.5 hours. The reaction mixture was cooled to −30°C. (2-PrOH, dry ice) and a solution of 6.3 g (0.033 mol) of 2,6-dichlorophenyl isocyanate in 80 ml of THF was added over a period of 2 hours. At the end of addition, the reaction mixture was allowed to warm to room temperature and stirring continued for 1 hour. The solids were filtered and the filtrate evaporated *in vacuo*. The residue was dissolved in dilute HCl, insolubles removed by filtration, and the aqueous solution washed with ether, made basic with cold 30% NaOH, and extracted with $CH_2Cl_2$. The combined organic layers were dried ($K_2CO_3$), filtered through a pad of diatomaceous earth and evaporated *in vacuo*. The residue was dissolved in MeOH, neutralized with ethereal HCl and treated with ether to give crystals which were recrystallized from MeOH/ether and then from a minimum amount of $H_2O$ to give pure *N*-(2,6-dichlorophenyl)-*N'*-(4,5,6,7-tetrahydro-1*H*-1,3-diazepin-2-yl)urea monohydrochloride hemihydrate; m.p. (175) 177—179°C dec.; UV max (MeOH) 244 (ε20,100) and 224 nm shld (ε20,800); IR (KBr) 3420, 3200, 1710, 1675, 1535 $cm^{-1}$.

ANAL.

| | |
|---|---|
| Calcd. for $C_{11}H_{14}Cl_2N_4O \cdot HCl \cdot O \cdot 5H_2O$: | C, 41.59; H, 4.65; N, 16.16; $H_2O$, 2.60. |
| Found: | C, 41.88; H, 4.70; N, 16.12; $H_2O$, 2.92. |

P. Stefanye and William L. Howard, *J. Am. Chem. Soc., 77,* 761 (1955) and Brit. Pat. 612,693, Nov. 16, 1948; *Chem. Abstr., 43,* 6670d (1949). Material dried *in vacuo* at 50 (5 mm Hz) 4 hours (to constant weight).

Example 10

According to the teaching of Example 9—B, the following compounds are prepared:

1. *N*-(2-Chloro-6-methylphenyl)-*N'*-(4,5,6,7-tetrahydro-1*H*-1,3-diazepin-2-yl)urea monohydrochloride; m.p. 159—161°C dec. 245—250°C.
2. *N*-(2,6-Dibromophenyl)-*N'*-(4,5,6,7-tetrahydro-1*H*-1,3-diazepin-2-yl)urea monohydrochloride and acid addition salts (solvates).
3. *N*-(2-Bromo-6-chlorophenyl)-*N'*-(4,5,6,7-tetrahydro-1*H*-1,3-diazepin-2-yl)urea monohydrochloride and acid addition salts (solvates).
4. *N*-(2-Bromo-6-methylphenyl)-*N'*-(4,5,6,7-tetrahydro-1*H*-1,3-diazepin-2-yl)urea monohydrochloride and acid addition salts (solvates).

The following examples illustrate the preparation of various pharmaceutical compositions of the present invention:

Example 11

1,000 hard gelatin capsules, each containing 200 milligrams of *N*-(2,6-dichlorophenyl)-*N'*-(4,5,6,7-tetrahydro-1*H*-1,3-diazepin-2-yl)urea are prepared from the following formulation:

| | Grams |
|---|---|
| *N*-(2,6-Dichlorophenyl)-*N'*-(4,5,6,7-tetrahydro-1*H*-1,3-diazepin-2-yl)urea | 200 |
| Starch | 250 |
| Lactose | 750 |
| Talc | 250 |
| Calcium stearate | 10 |

A uniform mixture of the ingredients is prepared by blending and employed to fill two-piece hard

gelatin capsules. The capsules are suitable to be orally administered to subjects with functional bowel disorders.

Example 12

Gelatin capsules are prepared as described in Example 11 except that in the formulation, 400 grams of *N*-(2,6-dibromophenyl)-*N'*-(4,5,6,7-tetrahydro-1*H*-1,3-diazepin-2-yl)urea is employed as active agent providing capsules containing 400 milligrams of *N*-(2,6-dibromophenyl)-*N'*-(4,5,6,7-tetrahydro-1*H*-1,3-diazepin-2-yl)urea.

Example 13

Gelatin capsules are prepared as described in Example VII except that *N*-(2,6-dibromophenyl)-*N'*-(4,5,6,7-tetrahydro-1*H*-1,3-diazepin-2-yl)urea is employed as the active agent.

Example 14

1,000 compressed tablets, each containing 500 milligrams of *N*-(2-chloro-6-methylphenyl)-*N'*-(4,5,6,7-tetrahydro-1*H*-1,3-diazepin-2-yl)urea are prepared from the following formulation:

| | Grams |
|---|---|
| *N*-(2-Chloro-6-methylphenyl)-*N'*-(4,5,6,7-tetrahydro-1*H*-1,3-diazepin-2-yl)urea | 500 |
| Starch | 750 |
| Dibasic calcium phosphate hydrous | 5,000 |
| Calcium stearate | 2.5 |

The finely powdered ingredients are mixed well and granulated with 10 per cent starch paste. The granulation is dried and compressed into tablets using starch as a disintegrant and calcium stearate as a lubricant.

**Claims**

1. An *N*-(phenyl)-*N'*-[(1,4,5,6-tetrahydropyrimidin-2-yl) or (4,5,6,7-tetrahydro-1-H-1,3-diazepin-2-yl)]urea having the formula:

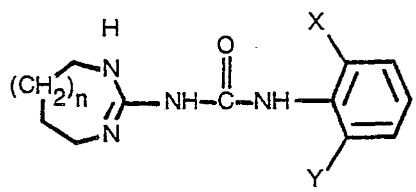

(I)

or pharmaceutically-acceptable salt thereof, wherein n is 0 or 1, X is Br or Cl and Y is Br, Cl or $CH_3$.

2. *N*-(2,6-Dichlorophenyl)-*N'*-(1,4,5,6-tetrahydropyrimidin-2-yl)urea and pharmaceutically-acceptable salts thereof.

3. *N*-(2-chloro-6-methylphenyl)-*N'*-(1,4,5,6-tetrahydropyrimidin-2-yl)urea and pharmaceutically-acceptable salts thereof.

4. *N*-(2,6-dichlorophenyl)-*N'*-(4,5,6,7-tetrahydro-1*H*-1,3-diazepin-2-yl)urea, and its pharmaceutically-acceptable salts.

5. *N*-(2,6-dibromophenyl)-*N'*-(4,5,6,7-tetrahydro-1*H*-1,3-diazepin-2-yl)urea, and its pharmaceutically-acceptable salts.

6. *N*-(2-chloro-6-methylphenyl)-*N'*-(4,5,6,7-tetrahydro-1*H*-1,3-diazepin-2-yl)urea, and its pharmaceutically-acceptable salts.

7. A pharmaceutical composition in dosage unit form, for treating hypertension, irritable bowel syndrome or diarrhea, comprising a compound according to any one of claims 1 to 6.

8. A process for preparing a compound according to any one of claims 1 to 6, characterised by reacting a compound of the formula:

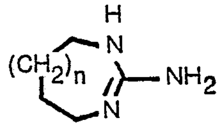

(II)

wherein n is as defined in claim 1, with a compound of the formula:

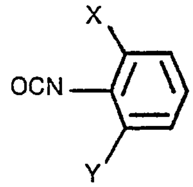

(III)

wherein X and Y are as defined in claim 1, in a suitable solvent, at temperatures between −20°C and 70°C, and, if desired, preparing a pharmaceutically-acceptable acid addition salt thereof.

9. A compound according to any one of claims 1 to 6, or a composition according to claim 7, for use in treating hypertension, irritable bowel syndrome, or diarrhea.

10. A process for preparing a composition in unit dosage form according to claim 7, comprising admixing a compound according to any one of claims 1 to 6 with a pharmaceutical carrier.

**Patentansprüche**

1. Ein N - (Phenyl) - N' - [(1,4,5,6 - tetrahydropyrimidin - 2 - yl)- oder (4,5,6,7 - tetrahydro - 1 - H - 1,3 - diazepin - 2 - yl)]harnstoff mit der Formel:

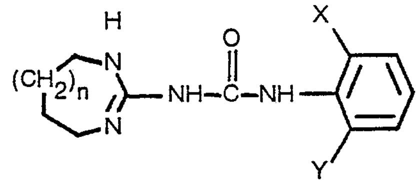

(I)

oder pharmazeutisch annehmbare Salze hievon, worin n den Wert 0 oder 1 aufweist, X für Br oder Cl steht und Y für Br, Cl oder $CH_3$ steht.

2. N-(2,6-Dichlorphenyl)-N'-(1,4,5,6-tetrahydropyrimidin-2-yl)harnstoff und pharmazeutisch annehmbare Salze hievon.

3. N-(2-Chlor-6-methylphenyl)-N'-(1,4,5,6-tetrahydropyrimidin-2-yl)harnstoff und pharmazeutisch annehmbare Salze hievon.

4. N-(2,6-Dichlorphenyl)-N'-(4,5,6,7-tetrahydro-1H-1,3-diazepin-2-yl)harnstoff und seine pharmazeutisch annehmbaren Salze.

5. N-(2,6-Dibromphenyl)-N-(4,5,6,7-tetrahydro-1H-1,3-diazepin-2-yl)harnstoff und seine pharmazeutisch annehmbaren Salze.

6. N-(2-Chlor-6-methylphenyl)-N'-(4,5,6,7-tetrahydro-1H-1,3-diazepin-2-yl)harnstoff und seine pharmazeutisch annehmbaren Salze.

7. Eine pharmazeutische Zusammensetzung in Dosiseinheitsform zur Behandlung von Bluthochdruck, Darmreizungssyndrom oder Durchfall, umfassend eine Verbindung gemäß einem der Ansprüche 1 bis 6.

8. Verfahren zur Herstellung einer Verbindung gemäß einem der Ansprüche 1 bis 6, dadurch gekennzeichnet, daß eine Verbindung der Formel:

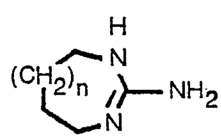

(II)

worin n wie in Anspruch 1 definiert ist, mit einer Verbindung der Formel:

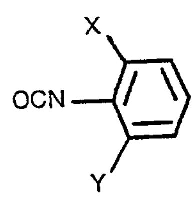

(III)

worin X und Y wie in Anspruch 1 definiert sind, in einem geeigneten Lösungsmittel bei Temperaturen zwischen −20°C und 70°C umgesetzt wird und gewünschtenfalls hievon ein pharmazeutisch annehmbares Säureadditionssalz bereitet wird.

9. Eine Verbindung nach einem der Ansprüche 1 bis 6, oder eine Zusammensetzung gemäß Anspruch 7, zur Verwendung in der Behandlung von Bluthochdruck, Darmreizungssyndrom oder Durchfall.

10. Verfahren zur Herstellung einer Zusammensetzung in Dosiseinheitsform gemäß Anspruch 7, umfassend das Vermischen einer Verbindung gemäß einem der Ansprüche 1 bis 6 mit einem pharmazeutischen Träger.

**Revendications**

1. *N*-(phényl)-*N'*-[(1,4,5,6-tétrahydropyrimidin-2-yl) ou (4,5,6,7-tétrahydro-1-H-1,3-diazépin-2-yl)]urée ayant pour formule:

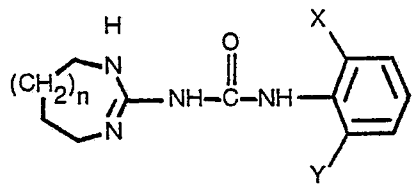

(I)

ou ses sels acceptables en pharmacie, où n est 0 ou 1, X est Br ou Cl et Y est Br, Cl ou $CH_3$.

2. *N*-(2,6-Dichlorophényl)-*N'*-(1,4,5,6-tétrahydropyrimidin-2-yl)urée et ses sels acceptables en pharmacie.

3. *N*-(2-chloro-6-méthylphényl)-*N'*-(1,4,5,6-tétrahydropyrimidin-2-yl)urée, et ses sels acceptables en pharmacie.

4. *N*-(2,6-dichlorophényl)-*N'*-(4,5,6,7-tétrahydro-1H-1,3-diazépin-2-yl)urée, et ses sels acceptables en pharmacie.

5. *N*-(2,6-dibromophényl)-*N'*-(4,5,6,7-tétrahydro-1*H*-1,3-diazépin-2-yl)urée, et ses sels acceptables en pharmacie.

6. *N*-(2-chloro-6-méthylphényl)-*N'*-(4,5,6,7-tétrahydro-1*H*-1,3-diazépin-2-yl)urée, et ses sels acceptables en pharmacie.

7. Composition pharmaceutique sous forme de dose unitaire pour le traitement de l'hypertension, du syndrome des intestins irritables ou de la diarrhée comprenant un composé selon l'une quelconque des revendications 1 à 6.

8. Procédé de préparation d'un composé selon l'une quelconque des revendications 1 à 6 caractérisé par la réaction d'un composé de formule:

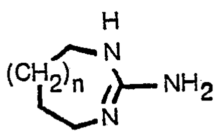

(II)

où n est tel que défini à la revendication 1 avec un composé de formule:

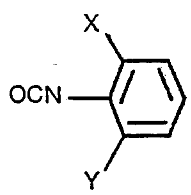

(III)

où X et Y sont tels que définis à la revendication 1, dans un solvant approprié, à des températures entre −20°C et 70°C et si on le souhaite, la préparation de son sel d'addition d'acide acceptable en pharmacie.

9. Composé selon l'une quelconque des revendications 1 à 6 ou composition selon la revendication 7 pour une utilisation pour le traitement de l'hypertension, du syndrome des intestins irritables ou de la diarrhée.

10. Procédé de préparation d'une composition sous forme de dose unitaire selon la revendication 7, consistant à mélanger un composé selon l'une quelconque des revendications 1 à 6 avec un support pharmaceutique.